# EUROPEAN PATENT APPLICATION

(11) **EP 1 892 299 A2**
(43) Date of publication of application: **27.02.2008**
(21) Application number: 07110021.8
(22) Date of filing: 18.09.1998
(51) Int. Cl.: C12N 15/67

(54) **Sense mRNA therapy**

(30) Priority: 19.09.1997 US 59371 P
(62) Divisional of application: 98946108.2
(71) Applicant: Sequitur, Inc., Natick, MA 01760 (US)
(72) Inventor: Woolf, Tod, Natick, MA 01760 (US)
(74) Representative: Williams, Richard Andrew Norman

(57) **Abstract**

Eukaryotic mRNA that encodes a protein of therapeutic interest is stabilised by at least one chemical modification. The stabilisation of the mRNA permits its use in sense RNA therapy wherein therapeutic proteins of interest are expressed transiently in cells. Modifications include 3' blocking, 5' blocking, a modified diguanosine (m7) cap linked to the mRNA by a chemically modified linkage, a 2' modified oligonucleotide, a poly A tail of greater than 50 bases in length, or a phosphorothiorate modification. Various disease states may be treated using such modified sense RNAs expressing relevant therapeutic proteins, including cystic fibrosis; muscular dystrophy; sickle cell anemia; thalasemia, coronary arterary disease, cancer, inflammation, stroke, heart disease, thrombosis, anemia, spinal-muscular atrophy, epilepsey, wound healing, septic shock, asthma, viral infection, bacterial or parasitic infection, diabetes, metabolic diseases, urea cycle disorders, Gaucher, schizophrenia, depression, Parkinson's disease, renal failure, liver failure, arthritis, impotence, baldness, pain, ulceration, and enlarged prostate.

## Description

### BACKGROUND

Gene therapy involves the introduction of heterologous DNA into a cell in order to modulate the expression of proteins. This can be done by introducing DNA into cells (see e.g., Malone. Focus (Life Technologies) 11, 61-66 (1989), Malone, et al. Proc. Natl. Acad. Sci. USA 86, 6077 (1989)). Other techniques utilize retroviruses, viruses that use RNA as their genetic material which is then reverse transcribed into DNA, to deliver heterologous nucleic acids to cells.

There are, however, several problems with the prior art methodologies of effecting protein expression. For example, heterologous DNA introduced into a cell can be inherited by daughter cells (whether or not the heterologous DNA has integrated into the chromosome) or by offspring. Introduced DNA can integrate into host cell genomic DNA at some frequency, resulting in alterations and/or damage to the host cell genomic DNA. Owing to these properties, DNA based gene therapy has inherent, potential adverse effects which can possible affect subsequent generations.

In addition, DNA must pass through several steps before a protein is made. Once inside the cell, DNA must be transported into the nucleus where it is transcribed into RNA. The RNA transcribed from DNA must then enter the cytoplasm where it is translated into protein. This need for processing creates long lag times before the appearance of the protein of interest.

Moreover, it is often difficult to obtain DNA expression in cells; frequently DNA enters cells but is not expressed. This can be a particular problem when DNA is introduced into cells *in vivo* or in is introduced into primary cell lines, exactly the types of cells which are the desired targets of gene therapy.

Technology, which would allow for the efficient expression of proteins of interest in a cell while eliminating these problems would be of tremendous benefit.

### SUMMARY

The present invention is directed to methods of altering eukaryotic, preferably mammalian, mRNA which result in its stabilization against nucleases and enable its use in sense RNA therapy to provide a protein of interest. The invention also pertains to compositions comprising such modified mRNAs and their methods of use. The modified RNAs of the present invention retain the ability to encode proteins.

In one aspect the invention pertains to modified, eukaryotic mRNA molecule encoding a therapeutically relevant protein, the mRNA molecule having a nucleotide sequence which comprises at least one chemical modification which renders the modified mRNA molecule stable, wherein the modified mRNA is translatable.

In one embodiment, the chemical modification comprises at least one end blocking modification. In one embodiment, the end blocking modification comprises the inclusion of a non-nucleotide blocking group blocking group. In another embodiment, the end blocking modification comprises the inclusion of a modified blocking group. In another embodiment, the end blocking modification comprises the inclusion of a 3' blocking modification. In another embodiment, the end blocking modification comprises the inclusion of a 5' blocking modification. In yet another embodiment, the 5' modification comprises the inclusion of a modified diguanosine (m7) cap being linked to the mRNA by a chemically modified linkage.

In another embodiment, the chemical modification comprises the inclusion of at least one modified nucleotide. In one embodiment, the modified nucleotide is selected from the group consisting of a 2' modified nucleotide and a phosphorothioate modified nucleotide.

In another aspect, the invention pertains to a modified, eukaryotic mRNA molecule encoding a therapeutically relevant protein, the mRNA molecule having a nucleotide sequence which comprises at least one modification which renders the modified mRNA molecule stable against nucleases, wherein the modification comprises the inclusion of a polyA tail of greater than about 50 bases in length, the mRNA molecule being translatable.

In another aspect, the invention pertains to a modified, eukaryotic mRNA molecule encoding a therapeutically relevant protein, the mRNA molecule having a nucleotide sequence which comprises at least one modification which renders the modified mRNA molecule stable against nucleases, wherein the modification of the mRNA molecule comprises complexing the mRNA with an agent to form an mRNA complex, the modified mRNA being translatable.

In one embodiment, the agent is a protein molecule. In a preferred embodiment, the protein is selected from the group consisting of: ribosomes, translational accessory protein, mRNA binding proteins, poly A binding proteins guanosine (7methyl) cap binding proteins, ribosomes, and translation initiation factors.

In one embodiment, the agent is a nucleic acid molecule. In one embodiment, the agent comprises a modification which increases the nuclease resistance of the mRNA molecule. In one embodiment, the agent comprises a chemical modification. In one embodiment the agent comprises a modification selected from the from the group consisting of: the inclusion of an end blocking group, the inclusion of a stabilizing sequence, the inclusion of a morpholino modification, the inclusion of a 2' modification, the inclusion of phosphoramidate modification, the inclusion of a phosphorothioate modification, and the inclusion of a poly A tail of at least about 50 nucleotides.

In another aspect, the invention pertains to a modified, eukaryotic mRNA molecule encoding a therapeutically relevant protein, the mRNA molecule having a nucleotide sequence which nucleotide sequence comprises at least one modification which renders the modified mRNA molecule stable against nucleases, wherein the modification comprises the depletion of Cytidines or Uridines from the nucleotide sequence, the modified mRNA being translatable.

In one embodiment, the Cytidines or Uridines are depleted from the 3' or 5' untranslated region of the mRNA molecule. In another embodiment, the Cytidines or Uridines are depleted from the coding region of the mRNA molecule.

In another aspect, the invention pertains to a modified, eukaryotic mRNA molecule encoding a therapeutically relevant protein, the mRNA molecule having a nucleotide sequence which nucleotide sequence comprises at least one modification which renders the modified mRNA molecule stable against nucleases, wherein the modification of the mRNA molecule comprises the incorporation of 3' or 5' sequences which naturally flank a second mRNA molecule which encodes a protein selected from the group consisting of: globin, actin GAPDH, tubulin, histone, and a citric acid cycle enzyme, the modified mRNA being translatable.

In another aspect, the invention pertains to a modified, eukaryotic mRNA molecule encoding a therapeutically relevant protein, the mRNA molecule having a nucleotide sequence which nucleotide sequence comprises at least one modification which renders the modified mRNA molecule stable against nucleases, wherein the modification of the mRNA molecule comprises the incorporation of an internal ribosome entry site selected from the group consisting of: a vascular endothelial growth factor IRES, encephalo myocardial virus IRES, a picornaviral IRES, a adenoassociated virus IREF, and a c-myc IRES.

In one embodiment the mRNA has a length of between about 500 to about 2000 nucleotides. In another embodiment, the mRNA has a length of between about 540 to about 1044 nucleotides.

In one embodiment, the modification comprises the inclusion of a sequence affecting the secondary structure of the mRNA, the sequence selected from the group consisting of: end G quartets psuedo knots; hairpins; and triple strand complexes.

In one embodiment, the subject mRNA molecules further comprise an intracellular delivery vehicle. In one embodiment, the delivery vehicle is selected from the group consisting of: cationic lipid containing complexes, uncharged lipids, nanoparticles.

In one embodiment, the mRNA encodes a signaling molecule selected from the group consisting of: a growth factor, a hormone, and a cytokine. In another embodiment, the mRNA encodes for a protein selected from the group consisting of: CFTR, distrophin, hemoglobin, fas ligand, basic FGF, p53, streptokinse, and urokinase.

In one embodiment, the mRNA molecule encodes an immunogen which causes an immune response in a subject.

In another embodiement, the mRNA molecule comprises the incorporation of 3' sequences which do not normally flank the mRNA molecule, an optimized Kozak translation initiation sequence, a coding region depleted of C's or U's, 5' sequences which do not normally flank the mRNA molecule, and a poly A tail of at least about 90 nucleotides in length.

In another aspect, the invention pertains to method of treating a disease state or disorder in a subject comprising administering an mRNA molecule to a subject such that the therapeutic protein is expressed in a cell of the subject and the disease state or disorder in the subject is treated.

In one embodiment, the disease state or disorder is selected from the group consisting of: cystic fibrosis; muscular dystrophy; sickle cell anemia; thalasemia, cancer, inflammation, thrombosis, anemia, spinal-muscular atrophy, viral infection, bacterial or parasitic infection, diabetes, Gaucher, and Parkinson's disease.

In another aspect, the invention pertains to a method of adding an exonuclease blocking group to an mRNA molecule comprising: enzymatically ligating an oligomer comprising an exonuclease blocking group to the mRNA molecule.

In another aspect, the invention pertains to a method of stabilizing an mRNA molecule which encodes a therapeutically relevant protein comprising: contacting an mRNA molecule with an agent such that a complex between the mRNA and the agent is formed such that the mRNA molecule is rendered resistant to nucleases, said mRNA molecule being translatable.

### BRIEF DESCRIPTION OF THE DRAWINGS

Figure 1 is a schematic which illustrates several methods of modifying mRNA to enhance its stability.
Figure 2 illustrates the expression of stabilized sense luciferase mRNA in cells.
Figure 3 illustrates that modified Renilla Luciferase mRNA is translated in cells.
Figure 4 illustrates that modified luciferase mRNA is translated in a mammalian cell-free system.
Figure 5 illustrates that modified Renilla luciferase mRNA is translated.
Figure 6 illustrates that modified Renilla luciferase mRNA is translated and illustrates direct transcription of luciferase from a template modified by PCR.
Figure 7 illustrates the effect of poly A tail length on mRNA stability
Figure 8 illustrates the use of splint ligation to add modified 3' and 5' ends to mRNA.

### DETAILED DESCRIPTION OF THE INVENTION

The present invention is based, at least in part, on improving the efficiency of RNA as a drug, by increasing the nuclease stability of the mRNA, while maintaining the ability of the mRNA to act as a template for translation. The invention also relates to mRNA therapy or sense mRNA therapy, i.e., the use of stabilized RNA as a drug in order to obtain the expression of a therapeutically relevant protein within a cell. The subject compositions and methods are useful in treating a myriad of disorders involving misexpression of proteins.

While *in vitro* transcribed messenger RNA, sense mRNA, can be transfected into cells, such RNA is readily and efficiently degraded by the cell, thus rendering it less effective than DNA for use in gene therapy. Moreover, RNA can be degraded even before it reaches a cell; RNA is extremely unstable in some bodily fluids, particularly in human serum. Thus, natural, unmodified mRNA can be degraded between the time it is administered to a subject and the time it enters a cell. Even within the cell, a natural mRNA decays with a half-life of between 30 minutes and several days. Although the modified sense mRNA of the invention is stabilized against nucleases, it retains the ability to be translated into protein. Thus, the present invention allows the transfection of RNA which has been stabilized against nucleases, instead of DNA, to direct the expression of a protein within a cell.

The instant methods and compositions have improved properties not found in the prior art approach of using DNA to bring about protein expression in cells. For example, as described in the instant examples, RNA is more readily expressed than DNA by certain types of cells. Moreover, in contrast to DNA, sense RNA is not genetically inherited, either by daughter cells or offspring, and it generally does not integrate into host cell genomic DNA. In addition, there is little or no danger of causing germ line mutations by introducing RNA into a host cell. Thus, while DNA based gene therapy can result in permanent alterations in the host cell genome, RNA based therapy is temporary. Accordingly, the use of RNA to bring about teh expression of a protein is more desirable as a medical therapy.

In addition, RNA requires fewer processing steps than DNA; it is readily translated once it enters the cytoplasm. (Malone. Focus (Life Technologies) 11, 61-66 (1989), Malone, et al. Proc. Natl. Acad. Sci. USA 86, 6077 (1989)). Thus, the instant sense RNA therapies have the advantage of directly delivering a template for translation into protein to the cytoplasm of a cell. In contrast to the use of DNA which requires several processing steps, the use of RNA to effect protein expression permits a sustained *in situ* translation of the protein of interest. This direct delivery of the template for translation means that when sense mRNA is used to bring about protein expression in a cell, the proteins are expressed much more rapidly than when DNA molecules are used for gene therapy. Thus, sense mRNA can be used to treat conditions which require an extremely rapid increase in the synthesis of a protein of interest, e.g., septic shock and other disease states which require immediate treatment).

Furthermore, the time course of protein expression in a cell can be more easily regulated when sense mRNA rather than DNA is introduced into cells. The stability of the sense mRNAs of the instant invention can be regulated by adjusting the type and/or amount of alteration made to the RNA molecule. By controlling the half-life of the mRNA molecule in this manner, the duration of protein expression can be controlled.

Before further description of the invention, the following definitions are, for convenience, collected here.

### L Definitions

As used herein, the term "modifed mRNA", includes a non naturally occurring sense messenger RNA that encodes a therapeutically relevant protein and that can be translated. As used herein, the term "modified" includes mRNA molecules which comprise at least one alteration which renders the mRNA molecule more resistant to nucleases than a naturally occurring, mRNA molecule encoding the same protein. Exemplary modifications to a nucleic acid sequence of an mRNA molecule which increase the stability of an mRNA molecule include, for example, the depletion of a base (e.g., by deletion or by the substitution of one nucleotide for another). Modifications also include the modification of a base, e.g., the chemical modification of a base. The term "chemical modifications" as used herein, includes modifications which introduce chemistries which differ from those seen in naturally occurring mRNA. For example, chemical modifications include covalent modifications such as the introduction of modified nucleotides, e.g.,nucleotide analogs, or the inclusion of pendant groups which are not naturally found in mRNA molecules.

In addition to modifications which include alterations in individual nucleotides of a codon of an mRNA molecule, the term "modification" also includes alteration of more than one nucleotide, e.g., a sequence of nucleotides. In addition, the term modification includes the addition of bases to a sequence (e.g., the inclusion of a poly A tail), alteration of the 3' or 5' ends of the mRNA molecule, complexing an mRNA molecule with an agent (e.g., a protein or a complementary nucleic acid molecule) as well as the inclusion of elements which change the structure of an mRNA molecule (e.g., which form secondary structures).

The term "therapeutically relevant protein" includes a protein that can be used in the treatment of a subject where the expression of a protein would be of benefit, e.g., in ameliorating the symptoms of a disease or disorder. For example, a therapeutically relevant protein can replace or augment protein expression in a cell which does not normally express a protein or which misexpresses a protein, e.g., a therapeutically relevant protein can compensate for a mutation by supplying a desirable protein. In addition, a "therapeutically relevant protein" can produce a beneficial outcome in a subject, e.g., can be used to produce a protein to which vaccinates a subject against an infectious disease.

As used herein, the term "stable" with regard to a sense mRNA molecule refers to enhanced resistance to degredation, e.g., by nucleases (endonucleases or exonucleases) which normally degrade RNA molecules. The stabilized mRNA molecules of the invention display longer half-lives than naturally occurring, unmodified mRNAs.

As used herein, the term "complexing" refers to forming a complex between a modified mRNA of the invention and an agent to form a complex of the mRNA molecule and the agent. The resulting complex renders the the mRNA molecule more stable. The term agent includes molecules, e.g., a protein or nucleic acid molecules which binds to the mRNA and protect it from nucleases.

As used herein, the term "end blocking modification" includes the incorporation of a non-nucleotide linkage (e.g., a propyl linker, such as is commercially available from TriLink Biotechnology of San Diego, CA) or modified nucleotide into a nucleotide sequence of an mRNA molecule such that nuclease degredation of the mRNAis reduced when compared to that seen in an unmodified mRNA molecule. End blocking modifications include both 3' and 5' modifications to an mRNA molecule.

As used herein the term "disease state or disorder" includes a condition which would benefit from the expression of a therapeutic protein (as described above), e.g, as demonstrated by a reduction in and/or an amelioration of symptoms.

### II. Sense mRNA Sequences For Stabilization

In one embodiment, the mRNA to be stabilized is eukaryotic in origin. Preferably the mRNA to be stabilized is mammalian in origin. In preferred embodiments, the subject sense mRNA molecules comprise characteristics of eukaryotic mRNAs, e.g., the presence of a 5'diguanosine (7m) cap, and/or the presence of a poly A tail.

mRNAs for stabilization using the instant methods can be isolated from cells, can be made from a DNA template, or can be chemically synthesized using methods known in the art prior to alteration using the instant methods. In preferred embodiment, mRNAs for modification are synthesized in vitro from a DNA template In one embodiment, the modified mRNAs of the invention are made using a high yield technology known in the art.

For stabilization using the described methods, sense mRNA molecules can be of any length. Preferably, sense mRNA molecules for stabilization are longer than about 50 nucleotides. In a preferred embodiment, sense mRNA sequences to be stabilized are longer than about 100 nucleotides. In another preferred embodiment, sense mRNA sequences are longer than about 250 nucleotides. In other preferred embodiments, the coding region of an mRNA molecule to be stabilized is between about 500 and about 1000 nucleotides in length. In another preferred embodiment, the coding region of an mRNA molecule to be stabilized is between about 1000 and 2000 nucleotides. In another preferred embodiment, the coding region of an mRNA molecule to be stabilized is between about 2000 and 3000 nucleotides. In another preferred embodiment, the coding region of an mRNA molecule to be stabilized is between about 3000 and 4000 nucleotides. In another preferred embodiment, the coding region of an mRNA molecule to be stabilized is between about 5000 and 6000 nucleotides. In another preferred embodiment, the coding region of an mRNA molecule to be stabilized is between about 6000 and 7000 nucleotides. In another preferred embodiment, the coding region of an mRNA molecule to be stabilized is between about 7000 and 8000 nucleotides.

### III Stabilization of Sense RNA sequences By Sequence Modification

Increasing the stability of mRNA molecules prior to their use in sense mRNA therapy can be accomplished in a number of ways. Alterations to the nucleotide sequences of mRNA molecules which result in increased stability of the mRNA molecules are described in detail in the following subsections.

### A. Base Depletion

### 1.Base Elimination.

In one embodiment of the invention, the number of Cytidines (C's) and /or Uridines (U's) in an mRNA sequence is reduced. While RNA containing C's and U's is rapidly degraded in serum, RNA devoid of C's and U's has been found to be stable to most RNases (Heidenreich, et al. J Biol Chem 269,2131-8 (1994). For example, while a short ribozyme RNA is degraded in serum in several minutes, such an RNA with 2' sugar protected C's and U's is very stable (T1/2 = 12 hours) (Heidenreich, et al. supra). Thus, reducing the number of C's and/or U's leads to more stable RNAs.

In one embodiment, the number of C's and/or U's can be reduced by deleting these nucleotides from an mRNA sequence. In one embodiment, C's and/or U's are eliminated from the coding region of an mRNA molecule. In another embodiment, the C's and U's are eliminated from the 5' untranslated region (UTR) and/ or the 3' UTR of an mRNA molecule. In a one embodiment of the invention, 100% of the C's and/or U's in of the nucleotide sequence of an mRNA molecule are depleted (e.g., eliminated or substituted). In another embodiment, at least about 90% of the C's and/or U's in the nucleotide sequence of an mRNA molecule are depleted. In yet another embodiment, at least about 80% of the C's and/or U's in the nucleotide sequence of an mRNA molecule are depleted. In still another embodiment, at least about 70% of the C's and/or U's in the nucleotide sequence of an mRNA molecule are depleted. In yet another embodiment, at least about 60% of the C's and/or U's in the nucleotide sequence of an mRNA molecule are depleted. In still another embodiment, at least about 50% of the C's and/or U's in the nucleotide sequence of an mRNA molecule are depleted. In another embodiment, at least about 40% of the C's and/or U's in the nucleotide sequence of an mRNA molecule are depleted. In another embodiment, at least about 30% of the C's and/or U's in the nucleotide sequence of an mRNA molecule are depleted. In another embodiment, at least about 20% of the C's and/or U's in the nucleotide sequence of an mRNA molecule are depleted. In another embodiment, at least about 10% of the C's and/or U's in the nucleotide sequence of an mRNA molecule are depleted.

In one embodiment, the length of the 5' and 3' UTR is reduced. In one embodiment, the 5' and/or 3' UTR is eliminated. In another embodiment the length of the 5' and/or 3' UTR is decreased from about 1 to about 50 nucleotides (not including the poly A tail length). This reduction in the length of the 5' and/or 3'UTR can further reduce RNA degradation, by reducing the length of RNA susceptible to RNase endonuclease cleavage. In certain embodiments, however, it may be desirable to leave C's and U's in a Kozak translation initiation sequence.

### 2. Base Substitution

In another embodiment, the number of C's and/or U's is reduced by substitution of one codon encoding a particular amino acid for another codon encoding the same or a related amino acid. This is particularly desirable when alterations are being made to the bases in the coding region of an mRNA sequence. Within the coding region of an mRNA, the constraints on reducing the number of C's and U's in a sequence will likely be greater than in an untranslated region of an mRNA, i.e., it will likely not be possible to eliminate all of the C's and U's present in the message and still retain the ability of the message to encode the desired amino acid sequence. However, the degeneracy of the genetic code should allow the number of C's and/or U's that are present in the wild-type sequence to be reduced, while maintaining the same coding capacity. Depending on which amino acid is encoded by a codon, several different possibilities for modification of RNA sequences may be possible. In the case of amino acids encoded by codons that comprise exclusively A or G, no modification would be necessary. For instance:
the codons for Glu (GAA or GAG) or Lys (AAA or AAG) would not require any alteration because no C's or U's are present.

In other cases, codons which comprise C's and/or U's can be altered by simply substituting other codons that encode the same amino acids but that do not comprise C and/or U. For instance:
the codons for Arg can be altered to AGA or AGG instead of CGU, CGC, CGA or CGG, or
the codons for Gly can be altered to GGA or GGG instead of GGU or GGC.

In other cases, rather than eliminating C's and/or U's from codons, the C and U content can be reduced by modifying a nucleic acid sequence of an mRNA to comprise codons that use fewer C's and/or U's. For instance:
Pro can be encoded by CCA or CCG instead of CCU or CCC
Thr can be encoded by ACA or ACG instead of ACU or ACC
Ala can be encoded by GCA or GCG sited of GCU or GCC
Leu can be encoded by CUA or CUG, UUA or UUG instead of CUU or CUC
Ile can be encoded by AUA instead of AUU and AUC
Val can be encoded by GUA or GUG instead of GUU or GUC
Ser can be encoded by AGU or AGC instead of UCU, UCC, UCA, or UCG

However, there are instances in which the C and/or U content of particular codons cannot be altered by sequence changes and still encode the same amino acid. For instance:
Met - AUG (no improvement possible)
Tyr - UAU or UAC (no improvement possible)
Stop - UAA, UAG or UGA (no improvement possible)
His - CAU or CAC (no improvement possible)
Gln - CAA or CAG (no improvement possible)
Asn - AAU or AAC (no improvement possible)
Asp - GAU or GAC (no improvement possible)
Cys - UGU or UGC (no improvement possible)
Trp - UGG (no improvement possible)
Phe - UUU or UUC (no improvement possible)

There are a variety of different methods that can be used to substitute codons that comprise C's and/or U's for those which comprise fewer C's and U's. For example, base substitutions can be made in the DNA template used for making an mRNA by standard site-directed mutagenesis (See, for example, Molecular Cloning A Laboratory Manual, 2nd Ed., ed. by Sambrook, Fritsch and Maniatis (Cold Spring Harbor Laboratory Press: 1989 or 1991 edition). In the case of short coding regions (e.g., coding regions which encode peptides),an entire mRNA can be synthesized chemically using standard techniques. In the case of chemically synthesized RNAs it may be desirable to make other modifications to further enhance mRNA stability. For example, a diguanosine (7m) cap (n7methylG(5')ppp(5')G) can be added to the synthetic RNA enzymatically (Theus and Liarakos. Biochromatography 9, 610-615 (1990), Nielsen and Shapiro. Nucleic Acids Research 14, 5936 (1986) or chemically during synthesis. Likewise, a poly A tail can be added enzymatically, e.g., with poly A polymerase (Yokoe and Meyer. Nature Biotechnology 14, 1252-1256 (1996)) or during chemical synthesis.

In other embodiments, the subject RNAs can be made more nuclease resistant by removing nuclease sensitive motifs, i.e., more nucleotides of an mRNA sequence, rather than by removing or substituting individual bases in a codon. Certain mRNAs are naturally unstable in a cell, and this is normally due to the existence of destabilizing sequence motifs within such unstable mRNAs that are recognized by nucleases such as those described by Brown et al. (1993. Genes Dev. 7:1620). If such sequences exist in a sense messenger RNA sequence of interest, they can be eliminated, replaced or modified by standard genetic engineering of the DNA transcription template (Molecular Cloning A Laboratory Manual, 2nd Ed., ed. by Sambrook, Fritsch and Maniatis (Cold Spring Harbor Laboratory Press: 1989 or1991 edition)).

### B. Chemical Modifications

In one embodiment an mRNA comprises a chemical modification which is a 2' modification (e.g., a 2' amino or a 2' sugar modification). In yet another embodiment, the number of C's and/or U's in the nucleotide sequence of an mRNA molecule can be reduced by incorporating analogs of C's and U's (as well as or in addition to incorporating other analogs or making other modifications). For example, 2' F-U and 2'F-C triphosphates can be incorporated into the subject sense mRNA molecules.

Since 2'F-U and 2'F-C are recognized by some enzymes and, thus, are very sterically similar to natural RNA, sense mRNA containing these modifications is suitable as a template for translation, to some degree (Nucleic Acids Res. 1994. 22:4963). 2' F-U and 2'F-C triphosphate can be incorporated into *in vitro* transcribed mRNA, since they are recognized by bacterial polymerases (Aurup, et al. Biochemistry 31, 9636-41 (1992)). 2' F-U and 2'F-C are remarkably stable against endoribonuclease. However, 2'F-U and 2'F-C triphosphates do not incorporate as well as natural triphosphates in *in vitro* transcription. Therefore, in preferred embodiments, the technique for reducing C and/or U content in the nucleotide sequence of an mRNA molecule is combined with the use of 2'F C and 2' F U during transcription to reduce the number of C's and U's which need to be chemically modified.

In another embodiment of the invention A's may be chemically modified to create 2'F A as described in the art (see e.g., Nucleic Acids Res. 1994. 22:4963).

In preferred embodiments, only a fraction of the nucleotides of an mRNA sequence are chemically modified (i.e., are replaced with analogs). For example, in one embodiment of the invention, 100% of the C's and/or U's in of the nucleotide sequence of an mRNA molecule are chemically modified. In another embodiment, at least about 90% of the C's and/or U's in the nucleotide sequence of an mRNA molecule are chemically modified. In yet another embodiment, at least about 80% of the C's and/or U's in the nucleotide sequence of an mRNA molecule are chemically modified. In still another embodiment, at least about 70% of the C's and/or U's in the nucleotide sequence of an mRNA molecule are chemically modified. In yet another embodiment, at least about 60% of the C's and/or U's in the nucleotide sequence of an mRNA molecule are chemically modified. In still another embodiment, at least about 50% of the C's and/or U's in the nucleotide sequence of an mRNA molecule are chemically modified. In another embodiment, at least about 40% of the C's and/or U's in the nucleotide sequence of an mRNA molecule are chemically modified. In another embodiment, at least about 30% of the C's and/or U's in the nucleotide sequence of an mRNA molecule are chemically modified. In another embodiment, at least about 20% of the C's and/or U's in the nucleotide sequence of an mRNA molecule are chemically modified. In another embodiment, at least about 10% of the C's and/or U's in the nucleotide sequence of an mRNA molecule are chemically modified.

In another embodiment of the invention, alpha phosphorothioate rNTP's can also be incorporated into an mRNA molecule during an *in vitro* transcription reaction, to create phosphorothioate RNA. Alternatively, alpha phosphorothioate CTP and UTP can be used to create partially phosphorothiaote RNA, such that not all of the C's and/or U's are replaced with analogs.

In addition to the modification of C's and/or U's in the mRNA sequence, in another embodiment, A's and/ or G's can be modified, i.e., replaced with analogs. For example, in a preferred embodiment, thioate is incorporated into the poly A tail of an mRNA molecule.

Phosphorothioate RNA can be made by simply adding the appropriate modified alpha thiotriphosphate nucleotide into the *in vitro* transcription reaction, using standard *in vitro* transcription conditions and commercially available alpha phosphorothioate monomers (Melton, 1988; Molecular Cloning A Laboratory Manual, 2nd Ed., ed. by Sambrook, Fritsch and Maniatis (Cold Spring Harbor Laboratory Press: 1989 or 1991 edition)). The phosphorothioate linkage is known to decrease nuclease degradation and is sterically and electrically very similar to natural RNA, so it can be recognized by the ribosome for translation. Any modified triphosphate nucleotide precursor which resists nucleases, and which is recognized by a purified RNA polymerase can be incorporated into an mRNA of the present invention to increase its nuclease stability (for example, UTP-biotin). In preferred embodiments, the resulting phosphorothioate mRNA is similar enough to natural mRNA that it is recognized by the translational machinery of the cell.

In another example of the incorporation of a nucleotide analog into an mRNA molecule, boranotriphosphates can be incorporated during transcription as thioates and 2'F. These analogs also act to chemically modify the mRNA and protect it against nucleases (He et al. Symposium on RNA Biology II: Tool & Target, Nucleic Acids Symposium Series No. 36. Oxford University Press, p. 159 (1997).; He et al. 1998. J. of Organic Chemistry 63:5769).

### 3' Blocking Groups

3' blocking groups include both the inclusion of modified nucleotide or non-nucleotide linkages into a nucleotide sequence of an mRNA molecule such that nuclease degredation of the mRNA is reduced when compared to that seen in an unmodified mRNA molecule.

In one embodiment of the invention, an optional additional 3' or 5' blocking group, or cap, may be added to an mRNA molecule to provide resistance to nucleases. This cap can be added enzymatically by a poly A polymerase, by ligation (using, for example, T4 RNA ligase), or by chemical methods (e.g., using the splint ligation method as described in Biochemie. 1994. 76:1235). A 3' or 5' end cap can also be made simply by designing a sense messenger RNA molecule such that the 3' or 5' end forms a highly stable secondary or tertiary structure, such as, e.g., a single or series of pseudoknots, triple strand complexes, G-quartet forming sequences, and or stable hairpins. For example, the following sequences would form secondary structures that would be predicted to block or partially block exonucleases.
An exemplary G Quartet forming sequence is illustrated by:
5'
GGGGGGGGGAAAAAGGGGGGGGGGAAAAAGGGGGGGGGGAAAAAGGGGG GGGGG
An exemplary hairpin forming sequence is illustrated by:
5' GGGGGGGGGGAAAAACCCCCCCCCC

In one embodiment, the 3' end of an mRNA molecule is modified to eliminate most or all of the 3' untranslated region, with the exception of the poly A tail.

### 5' Blocking Groups

The natural 5' diguanosine cap is also important in RNA stabilization (Molecular Cloning A Laboratory Manual, 2nd Ed., ed. by Sambrook, Fritsch and Maniatis (Cold Spring Harbor Laboratory Press: 1989 or 1991 edition)). The stability of the cap can be increased by the addition of phosphorothioate linkages to to any or all of the phosphates on the cap precursor during *in vitro* transcription as is known in the art (See, e.g., Nucleic Acids Research 1991. 19:547; Nucleic Acids Symposium Series. 1991. 125:151). Additionally or alternatively, the normal 2'-O-methyl modification of the diguanosine cap could be changed to the myria of 2' modified analogs known in the art (for example, 2'-O-ethyl, propyl, methoxyethoxy, allyl, or 2'F, or 2' amino). In some cases, the cap is added after *in vitro* transcription, and the modified stabilized cap may also be incorporated at this stage (see, e.g., Molecular Cloning A Laboratory Manual, 2nd Ed., ed. by Sambrook, Fritsch and Maniatis. Cold Spring Harbor Laboratory Press: 1989 or 1991 edition; Theus and Liarakos. Biochromatography 9, 610-615 (1990); Nielsen and Shapiro. Nucleic Acids Research 14,5936 (1986)). This modification may inhibit the decapping machinery in the cell.

When making alterations to a 5' cap, it is preferable to use modifications, like phosphorothioates or 2' F nucleosides, which have similar steric structures to their natural counterparts, so as to not adversely affect cap synthesis, or the role of the cap in translation initiation.

In another embodiment, the 5' end can be more drastically modified, for example with neutral methylphosphonate linkages, or 2'-O-Alkyl modified sugars (Lamond. Biochem. Soc. Transactions 21, 1-8 (1993), Blake, et al. Biochemistry 24, 6139-45 (1985)).

If such modified nucleic acids are not recognized by the translation machinery, an internal translation initiation sequence can be added to the mRNA sequence as described in more detail below.

### C. Increased Length of the poly A tail

In yet other embodiments of the invention, stabilization of mRNA can be accomplished by increasing length of the Poly A tail. The poly A tail is thought to stabilize natural messengers, and synthetic sense RNAs.

In one embodiment a long poly A tail can be added to a mRNA molecule, e.g., a synthetic RNA molecule, thus rendering the RNA even more stable. Poly A tails can be added using a variety of art-recognized techniques. For example, long poly A tails can be added to synthetic or *in vitro* transcribed RNA using poly A polymerase (Yokoe and Meyer. Nature Biotechnology 14, 1252-1256 (1996)). Long poly A tails can also be encoded by a transcription vector, however, long repeats can be unstable during bacterial propagation. The use of bacterial strains which comprise mutations in recombination enzymes (e.g., as commercially available from Stratagene) can reduce this potential problem. In addition, poly A tails can be added by transcription directly from PCR products, as described in the appended Examples. Poly A may also be ligated to the 3' end of a sense RNA with RNA ligase (see, e.g., Molecular Cloning A Laboratory Manual, 2nd Ed., ed. by Sambrook, Fritsch and Maniatis (Cold Spring Harbor Laboratory Press: 1989 or 1991 edition)). Modified poly A tails may also be added using a splint ligation method (Biochemie. 1994. 76:1235).

As mentioned above, phosphorothioate linkages, or other stabilizing modifications of RNA, may be incorporated into a poly A tail to add further stabilization to an mRNA molecule. Such modified Poly A tails may be added to sense mRNA molecules using poly A polymerase. In yet another embodiment, other, more drastically modified, analogs of A or other bases can be incorporated into a poly A tail (e.g., 2'-O-methyl or methylphosphonate modified). In one embodiment of the invention, other modifications may be made downstream of the poly A tail in order to retain the poly A binding sites and further block 3' exonucleases.

In one embodiment, the length of the poly A tail is at least about 90 nucleotides in another embodiment, the length of the poly A tail is at least about 200 nucleotides. In yet another embodiment, the length of the poly A tail is at least about 300 nucleotides. In still another embodiment, the length of the poly A tail is at least about 400 nucleotides. In yet another embodiment, the length of the poly A tail is at least about 500 nucleotides.

In one embodiment, the length of the poly A tail is adjusted to control the stability of a modified sense mRNA molecule of the invention and, thus, the transcription of protein. For example, since the length of the poly A tail can influence the half-life of a sense mRNA molecule the length of the poly A tail can be adjusted to control the level of resistance of the mRNA to nucleases and thereby control the time course of protein expression in a cell.

### D. Complexing mRNA with Agents

In yet another embodiment, the stability an mRNA and efficiency of translation may be increased by forming a complex of a naked mRNA molecule with proteins which normally complex with naturally occurring mRNAs within a cell (see e.g., U.S. patent 5,677,124). This can be accomplished by combining human poly A and a protein with the mRNA to be stabilized *in vitro* before complexing the mRNA with a delivery vehicle. Exemplary proteins include, translational accessory protein, mRNA binding proteins, and/or translation initiation factors. In yet another embodiment, polysomes can be formed *in vitro* and complexed with the delivery vehicle.

In preferred embodiments, the protein used to complex to a modified mRNA molecule of the invention is a eukaryotic protein, preferably a mammalian protein. In another preferred embodiment, the protein used to complex to a modified mRNA molecule of the invention is not a bacteriophage protein.

In yet another embodiment mRNA molecules for use in sense therapy can be - modified by hybridization to a second nucleic acid molecule. When RNA is hybridized to a complementary nucleic acid molecule (e.g., DNA or RNA) it is protected from many nucleases; the RNase protection assay (Krieg and Melton. Methods in Enzymology 155, 397-415 (1987)) is based on this finding. The stability of hybridized mRNA is likely due to the inherent single strand specificity of most RNases. Thus, when mRNA is in a duplex, the 2'-OH is sterically constrained from forming the structure in which this group acts a nucleophile to attach a phosphodiester backbone.

In preferred embodiments, mRNA is hybridized with an agent to protect sense mRNA from degradation before in reaches the cell.

Since if an entire mRNA molecule was hybridized to a complementary nucleic acid molecule it would likely interfere with translation initiation, in preferred embodiments the 5' untranslated region and the AUG start region of the mRNA molecule are left unhybridized, i.e., in single stranded form, such that translation can initiate. After translation is initiated, the unwinding activity of the ribosome complex can function even on high affinity duplexes (Liebhaber. J. Mol. Biol. 226, 2-13 (1992), Monia, et al. J Biol Chem 268, 14514-22 (1993)) so translation can proceed normally.

In one embodiment, nucleic acid molecules comprising unmodified, complementary nucleic acid sequences are used to hybridize to and protect an mRNA molecule. In preferred embodiments, a modified protecting chemistry is used to prevent the duplexes comprising mRNA from activating cellular enzymes. For example, if a DNA oligomer is used as the complementary nucleic acid molecule, the resulting DNA/RNA duplex could activate endogenous RNase H and result in the degredation of the sense mRNA (Monia, et al. J Biol Chem 268, 14514-22 (1993)). Likewise, if RNA is used as the complementary nucleic acid molecule, the resulting double stranded RNA molecule could be a substrate for the endogenous double stranded adenosine deaminase enzyme. RNA duplexes can be formed in trans or in cis, for example, by hybridizing the mRNA to a complementary RNA (trans) or designing the sequence of the RNA expression vector such that the reverse compliment of the part of the sense mRNA is encoded by sequences downstream of the sense mRNA. The resulting transcript would fold back on itself, forming e.g., a hairpin structure over the entire coding region (in cis). The endogenous double stranded adenosine deaminase could deaminate many of the A's in the mRNA and convert them to inosines, thus changing the coding capacity (Bass and Weintraub. Cell 55, 1089-1098 (1988), Woolf, et al. Proceedings of the National Academy of Science 92, 8298-302 (1995)).

In particularly preferred embodiments, the complementary nucleic acid molecules used to hybridize to and increase the stability of the sense mRNA molecules of the invention comprise one or more of the alterations described herein, e.g., comprise a 3' and 5' blocking groups.

In other particularly preferred embodiments, a nucleic acid molecule to be used to protect a sense mRNA molecule is modified by the addition of a protecting chemistry such that activation of cellular enzymes does not occur upon introduction of the double stranded nucleic acid molecule into a cell. Since a complementary nucleic acid which will hybridize to and protect the sense mRNA will not be translated, more options are available for modifying these complementary nucleic acids. For example, the altered backbone chemistries used in conjunction with antisense therapy, such as for example, morpholino modifications, 2' amino modifications, 2' amino sugar modifications, 2' F sugar modifications, 2'F modifications, 2' alkyl sugar modifications, uncharged backbone modifications, 2'-O-methyl modifications, or phosphoramidate, will be useful. Virtually all of these modifications fail to activate cellular enzymes (Wagner. Nat Med 1, 1116-8 (1995).

2'-O-methyl RNA is available commercially incorporated into oligomers (for example, it is available from Oligos Etc. of Wilsonville Oregon). 2'-O-methyl modified RNA has high hybrid affinity and is known to be unwound by translating ribosomes (Monia, et al. J Biol Chem 268, 14514-22 (1993)). Accordingly, it would be a good choice for a incorporating into an agent used to protect an mRNA molecule. Since most coding regions of mRNA molecules are relatively long, a series of tandem complementary 2'-O-methyl modified complementary oligomers can be designed to hybridize to the sense mRNA molecule downstream of the start AUG codon. These complementary nucleic acid molecules can hybridize to the entire coding region and, in some embodiments, to portions of the 3' untranslated region.

Alternatively, a longer protecting complementary oligomer may be made by *in vitro* transcription in the presence of the appropriately modified nucleoside triphosphate (Pagratis, et al. Nat Biotechnol 15, 68-73 (1997)). 2' amino and 2' fluoro nucleotide triphosphates can be incorporated into RNA by *in vitro* transcription (Pagratis, et al. Nat Biotechnol 15,68-73 (1997)). In other embodiments, 2'fluoro modified RNA, which does not form a substrate for RNase H when hybridized to unmodified RNA, may be used as a modification for a complementary nucleic acid molecule to protect sense mRNA.

In other embodiments, an mRNA molecule may be altered by including substituted purines or related analogs to increase resistance to nucleases such as adenosine deaminase. Adenosine deaminase converts adenosine to inosine by a deamination reaction: 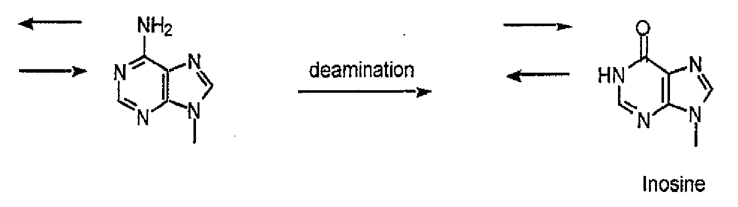 (the arrows in the following structures represent H-bond donator or acceptor).

Thus, for example, in one embodiment one or more A's bearing a 2-aminopurine substitution are incorporated into a modified sense mRNA molecule of the invention, e.g.,

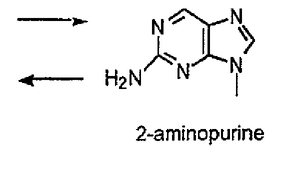

In another example, one or more A's in the nucleotide sequence of a modified sense mRNA molecule are substituted by 7-deaza adenosine (see e.g., U.S. Patent 5,594,121), 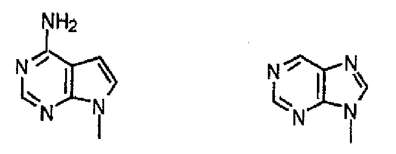 7- deaza adenosine

In yet another example, nebularin is incorporated (see, e.g, Kati et al. 1992. Biochemistry 31:7356).

In preferred embodiments a protecting agent (e.g., a nucleic acid, such as an oligomer or RNA molecule) is optionally modified by any of the modifications described herein for use in altering a sense mRNA molecule. For example, end-blocks, such as diguanosine caps, poly A tails, or further chemical modification, such as the addition of phosphorothioate (see e.g., WO 98/13526) can be used.

### E. Incorporation of 3' and/or 5' sequences

In one embodiment, an mRNA encoding a therapeutic protein can be modified by the incorporation 3' and/or 5' untranslated sequence which is not found flanking a naturally occurring mRNA which encodes the same therapeutic protein.

In one embodiment, 3' and/or 5' flanking sequence which naturally flanks an mRNA encoding a second, different protein (i.e., which is naturally found flanking mRNA encoding a protein unrelated to the protein encoded by the sense mRNA) can be incorporated into the nucleotide sequence of an mRNA molecule encoding a therapeutic protein in order to modify it. For example, 3' or 5' sequences from mRNA molecules which are stable (e.g., globin, actin, GAPDH, tubulin, histone, or citric acid cycle enzymes) can be incorporated into the 3' and/or 5' region of a sense mRNA molecule to increase the stability of the sense mRNA molecule.

Such 3' and/or 5' sequences can be added, for example, using the methods described in examples 4 and 5 below. Modified 5' and/or 3' untranslated sequences can be added using PCR to yield transcription templates with novel 5' and/or 3' ends. An exemplary T7 cloning oligo with the globin 5' UTR is: 
TAATACGACTCACTATAGGGAGGAGCTCACACTTGCTTTTGACACAACTGTG
TTTACTTGCAATCCCCCAAAACAGACACCATGGAAGACGCCAAAAACATAA
AGA
 An exemplary T7 3' cloning oligo with globin 3' UTR is:

ATCGGGTACCTTTTTTTTTTTTTTTTTTTTTTTTTTTTTTGCAATGAAAATAAAT
TTCCTTTATTAGCCAGAAGTCAGATGCTCAAGGGGCTTCATGATGTCCCCAT
AATTTTTGGCAGAGGGAAAAAGATCGCGGCCGCTTACAATTTGGACTTTCCG
CCCTTCT

In these examples the template coding region encodes luciferase, but this sequence could be substituted with any therapeutic relevant coding sequence using standard techniques.

In another embodiment, the subject sense mRNA molecules are additionally altered to include an internal ribosome entry site (IRES). The sequences required for maximal IRES activity have been described in the art (see e.g., Stein et al. 1998.. Mol. Cell. Biol. 18:3112; Gan et al. 1998. J. Biol. Chem. 273:5006; or Stonley et al. 1998. 16:423; Urabe et al. 1997. Gene 200:157). In preferred embodiments, an IRES is a vascular endothelial growth factor IRES, encephalo myocardial virus, a picornaviral IRES, a adenoassociated virus IREF, and a c-myc IRES. In particularly preferred embodiments, an IRES is less than about 500 bp in length. Preferably an IRES is less than about 400 bp in length. More preferably an IRES is less than about 300 bp in length. More preferably, an IRES is less than about 200 bp in length. More preferably, an IRES is about 150-200 bp in length, e.g., about 163 bp in length.

### F. Combinations of Alterations

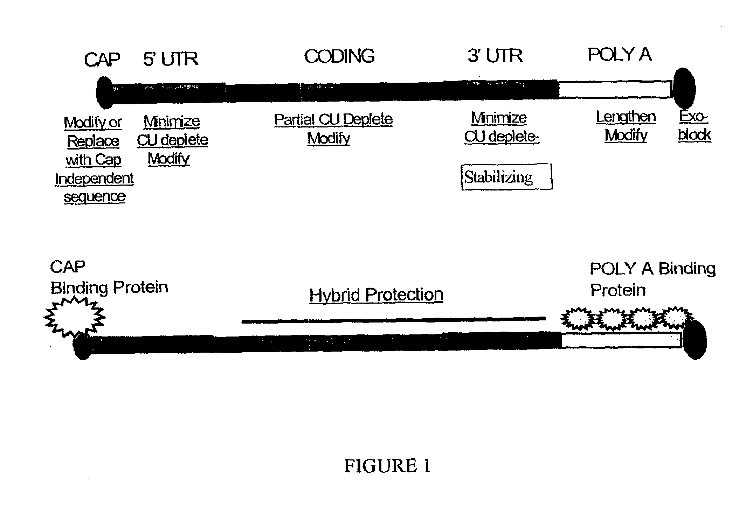

It will be understood that any of the above described methods for increasing the stability of mRNA molecules may be used alone or in combination with one or more of any of the other above-described methods to created an mRNA molecule which is optimally stabilized for use in the instant methods. For example, possible alterations to mRNA molecules are schematically illustrated in Figure 1.

In an exemplary embodiment, a modified sense mRNA of the invention comprises:
Sequences from the 5' end from a stable mRNA•an optimized Kozak translation initiation sequence•a coding region depleted of C's U's• sequences from the 3' UTR of a stable mRNA•a poly A tail of at least about 90 nucleotides in length.

In another exemplary embodiment, a modified sense mRNA of the invention comprises:
DNA containing mRNA (diguanosine Cap:5' UTR (modified or unmodified)•Coding region(modified or unmodified:3' UTR (modified or unmodified)•Poly A tail (modified or unmodified•DNA containing end block (for example phosphorothioate DNA, methylphosphonate DNA)
This construct can be prepared by ligation of the DNA containing end-block or splint ligation of the DNA containing end-block.

In another exemplary embodiment, a modified sense mRNA of the invention comprises:
A reduced pyridime 3' UTR•short example coding region (all histidine)• short pyridine depleted 3' UTR•and poly A tail (the cap can be added enzymatically, or during synthesis).
In another exemplary embodiment, a modified sense mRNA of the invention comprises:
An IRES from encephalo myocardial virus•with an adiitional 5' chemical blocking group.

### G. Testing for Increased Stabilization of mRNA

Sense mRNA which has been altered using any of the above methods can be tested for enhanced stability using techniques which are known in the art. For example, portions of the altered sense mRNA can be tested for destabilizing activity by cloning them into a stable mRNA (such as globin) and testing the effect on the stability of the globin mRNA (Brown et al., supra). In addition, the altered mRNA sequences can be transfected into cells and the level of protein produced can be measured and compared with unaltered mRNA sequences.

### IV. Administration of Stabilized Sense mRNA Molecules

The stabilized sense mRNA molecules of the invention can be administered to a subject using a variety of methods such that the stabilized mRNA molecules are delivered to a cell. For example, any of a number of delivery vehicles, e.g., cationic lipids, uncharged lipids, nanoparticles, or liposomes. In addition, the subject sense mRNA molecules can be directly injected into muscle. In one embodiment, biolistics can be used to deliver the mRNA molecules to a subject. In another embodiment peptoids can be used to deliver the subject mRNAs (Murphy et al. 1998. PNAS 95:1517; Huang et al. 1998. Chemistry and Biology 5:345). These methods are known in the art (see e.g., Malone. Focus (Life Technologies) 11, 61-66 (1989), Malone, et al. Proc. Natl. Acad. Sci. USA 86, 6077 (1989)).

Stabilized sense mRNA molecules can be used to treat any disease state in a subject which results from the lack of a functional protein, i.e., where the addition of a functional protein encoded by a stabilized sense mRNA would be of benefit to a subject. Exemplary disease states and disorders are described, e.g., in Harrisons Principles of Internal Medicine (13th Edition. 1994. Ed. By Isselbacher et al. McGraw Hill, NY. NY).

Exemplary disease states and disorders (and exemplary proteins which can be expressed to ameliorate symptoms in a subject) include: cystic fibrosis (CFTR), muscular dystrophy (dystrophin, utrophin), sickle cell anemia (hemoglobin), thalasemia (hemoglobin), coronary artery disease, cancer (recessive oncogenes, e.g., p53), inflammation (fas ligand or other immunoregulatory molecules e.g., soluble forms of endogenous receptors), stroke (basis FGF), heart disease, apoptosis, thrombosis (streptokinase or urokinase or TPA), anemia (EPO), spinal muscular atrophy (SMN), epilepsy, wound healing, psorisis, septic shock, asthma, viral infection (soluble receptors for viruses), bacterial or parasitic infections, diabetes (insulin), hypercholesterolemia, metabolic diseases, urea cycle disorders, gaucher (glucosylceramine-beta-glucosidase), schizophrenia, depression, Parkinson's (sonic hedgehog), renal failure, arthritis, impotence, baldness, pain, ulcerations, and enlarged prostate.

Once a disease state is identified, the sequence of the protein to be supplied (or the gene encoding the protein) can be determined. For known proteins, the sequence of the protein or the gene can be accessed using a database, such as GenBank. For example, Human EPO (GneBank Accession No.X02157), Human beta interferon: (GenBank Accession No. V00547, X04430), or Human cystic fibrosis transmembrane conductance regulator (CFTR) gene (GenBank Accession No.M28668) sonic hedgehog (GenBank Accession No.L38518), thrombopoeitin (GenBank Accession No.E12214), megakaryocyte growth and development factor(GenBank Accession No.U11025). Sense mRNA molecules encoding these proteins can be designed based on the protein sequence and using the genetic code or can be designed based on a DNA sequence.

The subject sense mRNA molecules can also be used to immunize against foreign proteins. For example, an mRNA encoding an immunogen can be administered to a subject in order to enhance the immune response to that immunogen.

The following invention is further illustrated by the following examples, which should not be construed as further limiting. The contents of all references, pending patent applications and published patents, cited throughout this application (including the "Background" Section) are hereby expressly incorporated by reference.

### EXAMPLES

### Example 1. Expression of stabilized sense mRNA

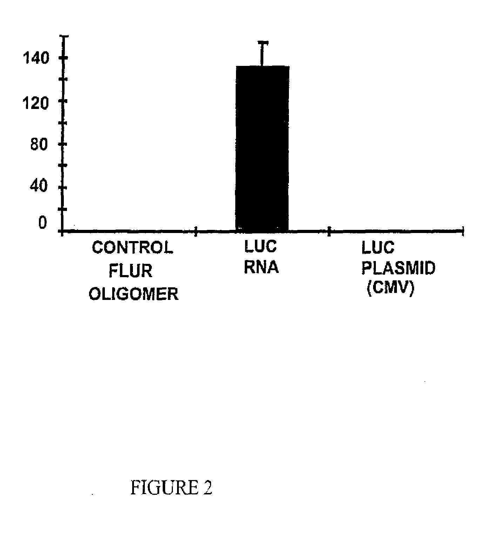

Stabilized sense mRNA was expressed in the bone-derived cell line MC-3T3 using mRNA transcript transfection. The results of mRNA transcript transfection were compared to those obtained using DNA plasmid transfection. Certain low passage bone cell lines, and many other primary cells and primary cell lines are resistant to transfection with DNA plasmids. Cells were transfected with a luciferase plasmid containing the CMV promoter (pRL-CMV) and a capped poly adenylated luciferase mRNA transcribed from an sP6 control template (Promega, Madison WI) using a Message Machine (commercially available from Ambion). Both plasmid and the mRNA were transfected at 1 µg/ml. The transfection was performed using lipofectin (5 ul/ml) according to the manufacturers protocol (Life Technology, Gaithersberg, MD) in the presence of Opti-MEM media (Life Technology, Gaithersberg, MD) for several hours. Cells were incubated in complete media for approximately 4 hours post-transfection, and were harvested and assayed for luciferase expression. A fluorescent oligomer served as a negative control for luciferase expression. Even at 24 hours post transfection, the level of luciferase expression was higher in the RNA transfection group than in the DNA plasmid transfection group (Figure 2).

### Example 2. Expression of diguanosine 5' G capped and 2'F cytidine containing Renilla Luc mRNA

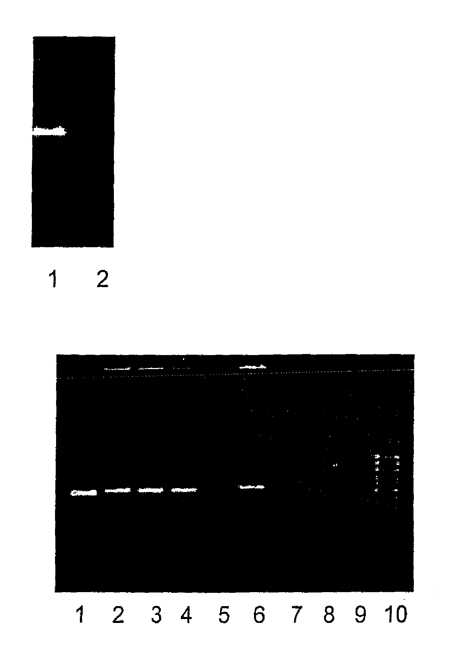

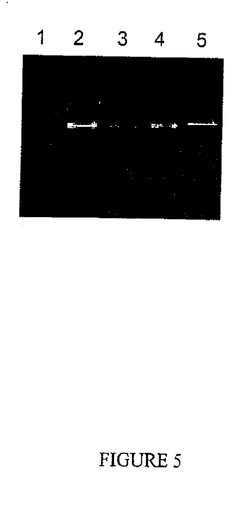

RNA was transcribed in the presence of certain alpha-phosphorothioate triphosphates, using Ambions Megascript in vitro transcription kit and T7 polymerase, using a linearized plasmid DNA template encoding firefly luciferase (T7 Control template, Promega, Madison WI). Transcripts were prepared in which alpha-thio nucleotides were substituted for nucleotide tirphosphates (NTPs). In groups 1-4, of Figure 3 each rNTP was substituted one at a time. Group 5 was transcribed completely with alpha-thio rNTPs. Group 6 was transcribed using unmodified rNTPs. Group 7 was transcribed from a mixture of alpha-thio and unmodified rNTPs. Figure 5 shows transcription in all of the groups, with the transcription in groups 1-4 being as high as in the unmodified control group (Figure 3).

### Example 3. Translation of Phosphorothioate containing mRNA

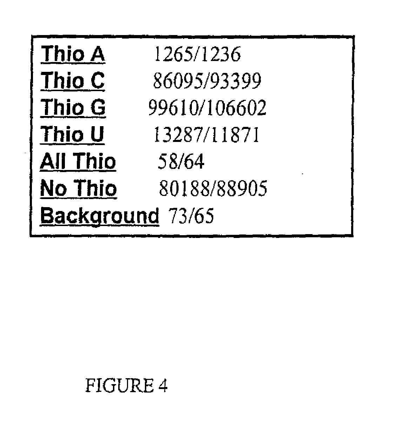

Sense mRNA encoding luciferase (luc) was transcribed in the presence of certain alpha-phosphorothioate triphosphates, using a Megascript in vitro transcription kit (commercially available from Ambion) and T7 polymerase, using a linearized plasmid DNA template encoding firefly luciferase (T7 Control template, Promega, Madison WT). Transcripts were prepared in which alpha-thio nucleotides were substituted for rNTPs. In transcripts 1-4, each rNTP was substituted one at a time (see Figure 4 ThioA, Thio C, Thio G, and Thio U groups). Transcript 5 was transcribed completely with alpha-thio rNTPs (see Figure 4, all Thio group). Transcript 6 was transcribed using unmodified rNTPs (see Figure 4, No Thio group). Transcript 7 was transcribed from a mixture of alpha-thio and unmodified rNTPs. This transcript contains a poly A tail (see Figure 4, background group). In the assay, 0.6 ug of each of the above transcripts was mixed with reticulocyte lysate cocktail and was incubated for 60 minutes. One µl of the each lysate reaction was then added to 100 µl of luciferin to assay the luciferase activity. The numbers shown in Figure 4 for each of the different groups represent duplicate readings of the same sample. Note that the comparatively low translation seen with thio U and thio A containing RNA in this cell-free system represents the minimum amount of translation one might expect, as the contaminating or free thio U and thio A nucleotides may inhibition translation.

Phosphorothioate containing luciferase mRNA has also been found to translate in cells.

### Example 4. Expression of Dimethyl G 5' capped phosphorothioate containing mRNA.

RNA was transcribed in the presence of certain alpha-phosphorothioate triphosphates using a Message Machine in vitro transcription kit (commercially available from Ambion) and T7 polymerase. Message was transcribed using a linearized plasmid template encoding Renila luciferase (commercially available from Promega, Madison, WI). Phosphorothioate ribonucleotide triphosphates were substituted as indicated. The resulting transcripts are expressed (see Figure 5) and since they have diguanosine caps, are particularly suitable for expression in mammalian cells.

### Example 5. Adding phosphorothioate and unmodified poly A tail to capped mRNA encoding luciferase using poly A polymerase

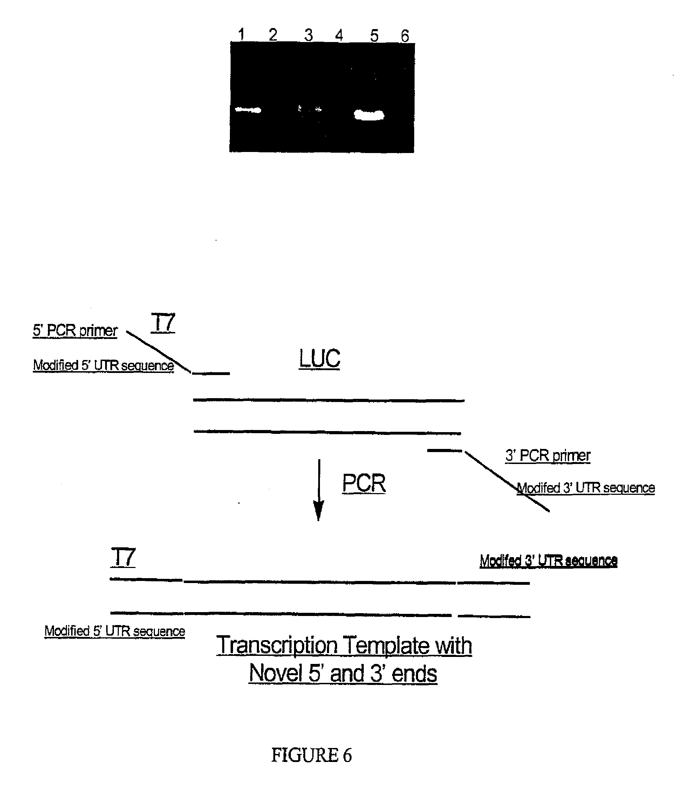

Using a linearized sp6 control luciferase PCR template (Promega, Madison WI), a transcription template was generated using a 5' and 3' primer. Several different 3' primers were employed, each containing 30,60, 90 or 120 nucleotide stretches or T's, which encoded for 30,60,90 or 120 nucleotide poly A tails, respectively. The 5' primer contained the T7 promotor sequence, and thus the PCR product was a linear template for in vitro transcription. (5' taa tac gac tea cta tag gga gga age tta cgc acg egg ccg cat cta gag (without upstream starts). (alternatively, a sequence without upstream AUG's can be used, as follows, 5'-taatacgactcactatagggaggaagettatgcatgcggccgcatctag). The templates were transcribed using a Message Machine (commercially available from Ambion) to produce diguanosine capped luciferase messages with poly A tails (Figure 6).

### Example 6. Production of mRNA containing poly A tails using a DNA template modified by PCR.

For each 500 µl of PCR reaction: 2 µg of each 100 mer primer (adjusted up or down according to length. i.e. 2 µg/100 mer, 1 µg/50 mer); 30 ng of linearized template; 50ul 10X Taq buffer GIBCO; 50ul dATCG mix (2mM stock, 200uM final conc.); 15 µl MgCl₂ (supplied with kit from GIBCO); 362 µl of water; 10µl of Taq polymerase; 3 µl of Template (10 ng / µL stock); 5 µl of 5' Primer (13 µM stock, 0.13 µM final conc.); 5 µl of 3' Primer (13 µM stock, 0.13 µM final conc.). The mixture was placed in 10 tubes each containing 50 µl of PCR reaction (for 1000 µL PCR reaction aliquot into 100 µL)

The PCR protocol used was: Initial denature: 94 degree 1.5 min; Denature: 94 degrees 1.5 min; Anneal: 40 degrees 2.5 min; polymerase: 72 degrees 3 min; Cycle 20 times; Final polymerase. 72 degrees 10 min; Additional incubation 37 degrees 10 min.

After the completion of the PCR program, 18 µL of reaction mixture was removed to check product on agarose gel. The RNA was Phenol extract 2X (with 1 volume of buffered phenol/CHCl3) and EtOH precipitated.

### Example 7. Poly A tails enhance protein expression.

Poly A tails can be added to RNA encoding luciferase (luc) by incubation with ATP (or aS ATP) and poly A polymerase. Procedure 1 or procedure 2 of the following protocol was used:

| **Polyadenylation protocol** | | |
|---|---|---|
| | **Procedure 1** | **Procedure 2** |
| | 1 mM ATP | 1 mM ATP rluc |

| rluc | | |
|---|---|---|
| | 35 ul H2O | 31 ul H2O |
| | 4 ul 1M Tris (40mM final) | 4 ul 1M Tris (40mM final) |
| | 10 ul 100 mM MgCl2 | 10 ul 100 mM MgCl2 |
| | 10 ul 25 mM MnCl2 | 10 ul 25 mM MnCl2 |
| | 5 ul 5M | 5 ul 5M NaCl |
| | NaCl | |
| | 1 100 mM ATP 1 mM final | 5 20 mM aS ATP 1 mM final |
| | 10 BSA | 10 BSA |
| | 10 RNA (rluc) 30ug | 10 RNA (rluc) 30 ug |
| | 2 ul RNase inhibitor | 2 ul RNase inhibitor |
| | 10 ul 100 mM DTT | 10 ul 100 mM DTT |
| | 3 ul (12 units polyA polymerase) | 3 ul (12 units polyA polymerase) |
| | **100** ul total volume | **100** ul total volume |

The reaction mixture from procedure 1 or procedure 2 was incubated at 37 for 30 minutes or 90 minutes. After completion of the reaction, 20 ug of glycogen was added as a carrier, along with 1/10 volume of ammonium acetate and 3 volumes of 100% ethanol (DEPC treated). The samples were incubated at -20 degrees for 1 hour or 2 hours. RNA was pelleted by centrifugation (12000g for 15 min). The supernatant was decanted and the pellet was washed with 1 ml of 75% EtOH. The supernatant was decanted and the the pellets were air dried. The pellets were then dissolved in 25 ul of DEPC treated water. The amount of mRNA was quantitated by absorbance at 260nm. The integrity and size of the samples was tested on a 1% denaturing agarose gel. The samples were found to be intact and the addition of ATP (unmodified) was successful.

Hela cells were plated at 1x 105/well in a 12 well plate. The following day, cells were transfected one of three different in vitro transcribed RNAs: rluc with no poly A tail, rluc with poly adenylation for 30 minutes with ATP, rluc with poly adenylation for 30 minutes with a-s ATP. Before incubation with lipofectin, the cells were rinsed with Opti-MEM to remove residual serum (900 µl of Opti-MEM was added per well). After 15 minutes, 100 ul of 10X lipofectin/RNA complex was added to the cells. The cells were transfected with 3 µl of RNA using lipofectin at a final concentration of 1 µg/ml. The cells were incubated for 5 hours at 37 degrees. Reporter lysis buffer (300 ul) was added to each well and cells were stored at -70oC overnight.

Luciferase activity was determined using a dual luciferase assay kit (commercially available from Promega; Madison, WI) and a luminometer (Lumicount from Packard Instument Company).

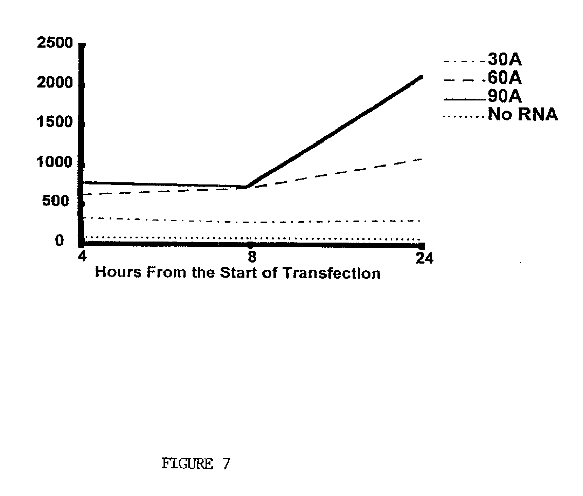

Sense mRNAs comprising poly A tails of about 60-90 A's in length were found to give higher levels of luciferase expression than mRNAs with no or a shorter poly A tail (see figure 7).

### Example 8. Preparation of sense mRNA molecules with exonuclease blocking groups

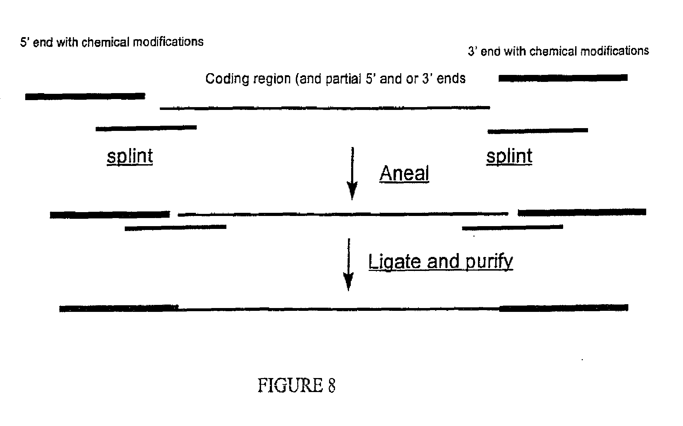

Oligomers with exonuclease blocking groups at the 3' and or 5' ends can be made e.g., by standard ligation with phage RNA ligases, or by splint ligations (e.g., Biochimie. 1994. 76:1235) or by-peptide fusions for the in vitro selection of peptides and proteins.Roberts RW, Szostak JW Proc Natl Acad Sci USA 1997 Nov 11 94:23 12297-302. Many exocuclease blocking groups are known in the art (WO9813526,THREE COMPONENT CHIMERIC ANTISENSE OLIGONUCLEOTIDES). A syntheisized end block like one of more multiple propyl linkers, are available e.g., from TriLink biotechnology, San Diego, CA)) A chemically modified 3' end with splint ligation can be made as follows:
An mRNA 3' end is created by ligating the parent mRNA 3' end (shown in lowercase, top row, to the synthetic 3' end containing a poly A tail (optionally) and a modified chemistry that forms a block to exonucleases (P=propyl linkers, Trilink Biotechnology, San Diego, CA). The optional splint oligomer guides the two termini together during the ligation, and increases the ligation efficiency. (see, e.g., Biochimie. 1994. 76:1235)-peptide fusions for the in vitro selection of peptides and proteins.Roberts RW, Szostak JW Proc Natl Acad Sci USA 1997 Nov 11 94:23 12297-302).
mRNA 5'-3'
aucgggccaauuuacagcauUUAGGCCUGAUCCGGAAAAAAAAAAAAAAAAAAAAAAAAAAAAPPPPPPP ggttaaatgtcgtaaatccggactaggcc Splint 3'-5']
This technique is illustrated in Figure 8.

### Example 9. Increasing stability of an mRNA molecule by pre-forming translation complexes

An mRNA molecule can be stabilized by forming translation complexes, for example, using the following protocol. Human poly A and cap binding protein are added to the mRNA *in vitro* before complexing with delivery vehicle. This can be accomplished using, e.g., purified recombinant poly A binding protein, or poly A binding protein purified from tissues. An *in vitro* complexing reaction simply involves combing the poly A binding protein with a synthetic mRNA that contains a poly A tail in physiological salts (e.g., 2mM Tris pH 8.0 and 50-300mM salts (KCl or NaCl). If desired, uncomplexed poly A binding protein can be removed by any one of a number commercially available native RNA isolation techniques. The complex is then added to an intracellular delivery vehicle, such as liposomes.

In another embodiment polysomes are formed *in vitro* and the intact polysomes are complexed with the delivery vehicle. Once the polysome is formed using standard conditions such as those used in reticulocyte lysates (Molecular Cloning A Laboratory Manual, 2nd Ed., ed. by Sambrook, Fritsch and Maniatis (Cold Spring Harbor Laboratory Press: 1989 or 1991 edition)). The polysomes may be stabilized using low temperature or salt conditions that are taught in the art, e.g., conditions used for polysome purification. The polysomes can also be further purified on a glycerol gradient or by using commercially available antibodies to the cap binding protein. The polysomes can then be added to an intracellular delivery vehicle, such as liposomes.

### Example 10. Protecting an mRNA molecule by hybridization

Standard procedures can be used for hybridizing nucleic acids *in vitro* (Molecular Cloning A Laboratory Manual, 2nd Ed., ed. by Sambrook, Fritsch and Maniatis (Cold Spring Harbor Laboratory Press: 1989 or 1991 edition)) can be used. For example, synthetic protecting nucleic acids can be mixed at 1:1 or greater molar ratios with the mRNA that is to be protected in salt buffer (for example, 400nM KCl or NaCl and 20mM tris. pH 7.5). The sample is heated to greater than 65 degrees centigrade for 2-10 minutes to denature secondary structure, then allowed to hybridize at approximately 15 degrees below the hybrid melting temperature (roughly 50-60 degrees centigrade) for one hour ( Molecular Cloning A Laboratory Manual, 2nd Ed., ed. by Sambrook, Fritsch and Maniatis (Cold Spring Harbor Laboratory Press: 1989 or 1991)). After hybridization, the excess protecting oligomer can be removed, or the complex can be added to an intracellular delivery vehicle, such as a liposome.

### EQUIVALENTS

Those skilled in the art will recognize, or be able to ascertain, using no more than routine experimentation, numerous equivalents to the specific substances and procedures described herein. Such equivalents are considered to be within the scope of this invention, and are covered by the following claims.

## Claims

1. A modified, eukaryotic mRNA molecule encoding a therapeutically relevant protein, said mRNA molecule having a nucleotide sequence which comprises at least one chemical modification which renders the modified mRNA molecule stable, wherein the modified mRNA is translatable.

2. An mRNA molecule as claimed in claim 1, wherein the chemical modification comprises at least one end blocking modification; optionally
(a) wherein the end blocking modification comprises the inclusion of a non-nucleotide blocking group and the inclusion of a modified nucleotide blocking group; or
(b) wherein the end blocking modification comprises the inclusion of a non-nucleotide blocking group and the inclusion of a non-nucleotide blocking group; or
(c) wherein the end blocking modification comprises the inclusion of a 3' blocking modification; or
(d) wherein the end blocking modification comprises the inclusion of a 5' blocking modification; preferably wherein the 5' modification comprises the inclusion of a modified diguanosine (m7) cap being linked to the mRNA by a chemically modified linkage.

3. An mRNA molecule as claimed in claim 1, wherein
(a) the chemical modification comprises the inclusion of at least one modified nucleotide; or
(b) the modified nucleotide is selected from the group consisting of a 2' modified nucleotide and a phosphorothioate modified nucleotide.

4. An mRNA molecule as claimed in claim 1, wherein the mRNA has a length of
(a) between about 500 to about 2000 nucleotides; or
(b) between about 500 to about 1000 nucleotides.

5. An mRNA molecule as claimed in claim 1, wherein said modification comprises the inclusion of a sequence affecting the secondary structure of the mRNA, said sequence selected from the group consisting of: end G quartets pseudo knots; hairpins; and triple strand complexes.

6. An mRNA molecule as claimed in claim 1, further comprising an intracellular delivery vehicle; preferably wherein said delivery vehicle is selected from the group consisting of: cationic lipid containing complexes, uncharged lipids, nanoparticles.

7. An mRNA molecule as claimed in claim 1, wherein said mRNA encodes
(a) a signalling molecule selected from the group consisting of: a growth factor, a hormone, and a cytokine; or
(b) a protein selected from the group consisting of: CFTR, distrophin, hemoglobin, fas ligand, basic FGF, p53, streptokinse, urokinase; or
(c) an immunogen which causes an immune response in a subject.

8. An mRNA molecule as claimed in claim 1, wherein said mRNA molecule comprises the incorporation of 3' or 5' sequences which do not normally flank said mRNA molecule, an optimized Kozak translation initiation sequence, a coding region depleted of C's or U's, and a poly A tail of at least about 90 nucleotides in length.

9. A modified, eukaryotic mRNA molecule encoding a therapeutically relevant protein, said mRNA molecule having a nucleotide sequence which comprises at least one modification which renders the modified mRNA molecule stable against nucleases, wherein the modification comprises the inclusion of a poly A tail of greater than about 50 bases in length, said mRNA molecule being translatable.

10. A modified, eukaryotic mRNA molecule encoding a therapeutically relevant protein, said mRNA molecule having a nucleotide sequence which nucleotide sequence comprises at least one modification which renders the modified mRNA molecule stable against nucleases, wherein the modification of the mRNA molecule comprises complexing the mRNA with an agent to form an mRNA complex, said modified mRNA being translatable.

11. An mRNA molecule as claimed in claim 10, wherein the agent is a protein molecule; optionally wherein said protein is selected from the group consisting of: ribosomes, translational accessory protein, mRNA binding proteins, poly A binding proteins dimguanosine (7m) cap binding proteins, ribosomes, and translation initiation factors.

12. An mRNA molecule as claimed in claim 10, wherein the agent is a nucleic acid molecule.

13. An mRNA molecule as claimed in claim 12, wherein the agent comprises a modification which increases the nuclease resistance of the mRNA molecule; optionally wherein the modification comprises a chemical modification, preferably wherein the agent comprises a modification selected from the group consisting of: the inclusion of an end blocking group, the inclusion of a stabilizing sequence, the inclusion of a morpholino modification, the inclusion of a 2' modification, the inclusion of phosphoramidate modification, the inclusion of a phosphorothioate modification, and the inclusion of a poly A tail of at least about 50 nucleotides.

14. A modified, eukaryotic mRNA molecule encoding a therapeutically relevant protein, said mRNA molecule having a nucleotide sequence which nucleotide sequence comprises at least one modification which renders the modified mRNA molecule stable against nucleases, wherein the modification comprises the depletion of Cytidines or Uridines from said nucleotide sequence, said modified mRNA being translatable.

15. An mRNA molecule as claimed in claim 14, wherein
(a) the Cytidines or Uridines are depleted from the 3' or 5' untranslated region of the mRNA molecule; or
(b) the Cytidines or Uridines are depleted from the coding region of the mRNA molecule.

16. A modified, eukaryotic mRNA molecule encoding a therapeutically relevant protein, said mRNA molecule having a nucleotide sequence which nucleotide sequence comprises at least one modification which renders the modified mRNA molecule stable against nucleases, wherein the modification of the mRNA molecule comprises the incorporation of 3' or 5' sequences which naturally flank a second mRNA molecule which encodes a protein selected from the group consisting of: globin, actin GAPDH, tubulin, histone, and a citric acid cycle enzyme, the modified mRNA being stable.

17. A modified, eukaryotic mRNA molecule encoding a therapeutically relevant protein, said mRNA molecule having a nucleotide sequence which nucleotide sequence comprises at least one modification which renders the modified mRNA molecule stable against nucleases, wherein the modification of the mRNA molecule comprises the incorporation of an internal ribosome entry site selected from the group consisting of: a vascular endothelial growth factor IRES, encephalo myocardial virus IRES, a picornaviral IRES, a adenoassociated virus IREF, and a c-myc IRES.

18. The use of an mRNA molecule of any of claims 1 to 17 for the manufacture of a medicament for administration to a subject such that the therapeutic protein is expressed in a cell of the subject and a disease state in the subject is treated.

19. A use as claimed in claim 18, wherein the disease state is selected from the group consisting of: cystic fibrosis; muscular dystrophy; sickle cell anemia; thalasemia, coronary arterary disease, cancer, inflammation, stroke, heart disease, thrombosis, anemia, spinal-muscular atrophy, epilepsey, wound healing, septic shock, asthma, viral infection, bacterial or parasitic infection, diabetes, metabolic diseases, urea cycle disorders, Gaucher, schizophrenia, depression, Parkinson's disease, renal failure, liver failure, arthritis, impotence, baldness, pain, ulceration, and enlarged prostate.

20. A method of adding an exonuclease blocking group to an mRNA molecule comprising: an enzyme to ligate an oligomer comprising said exonuclease blocking group to said mRNA molecule.

21. A method of stabilizing an messenger RNA molecule which encodes a therapeutically relevant protein comprising: forming a complex between the mRNA molecule and an agent such that said mRNA molecule is rendered resistant to nucleases, wherein said mRNA molecule is translatable.
